# EUROPÄISCHE PATENTSCHRIFT

(11) **EP 1 440 085 B1**
(45) Veröffentlichungstag und Bekanntmachung des Hinweises auf die Patenterteilung: **14.04.2010**
(21) Anmeldenummer: 02785166.6
(22) Anmeldetag: 07.10.2002
(51) Int. Cl.: C07K 14/035

(54) **TRANSIENTE IMMORTALISIERUNG VON ZELLEN DURCH ONKOGENPROTEINE ODER TELOMERPROTEINE**
TRANSIENT IMMORTALIZATION OF CELLS BY ONCOGENIC PROTEINS OR TELOMERASE
IMMORTALISATION TRANSITOIRE DE CELLUSES PAR DES PROTEINES ONCOGENIQUES OU TELOMERASE

(30) Priorität: 18.10.2001 DE 10152972
(43) Veröffentlichungstag der Anmeldung: 28.07.2004
(73) Patentinhaber: Heart BioSystems GmbH, 69120 Heidelberg (DE)
(72) Erfinder: KÜPER, Jan-Heiner, 47574 Goch (DE); MEYER, Ralph, 55596 Waldböckelheim (DE); MEYER-FICCA, Mirella, 55596 Waldböckelheim (DE); KUHN, Anne, 72072 Tübingen (DE)
(74) Vertreter: Simandi, Claus
(86) Internationale Anmeldenummer: PCT/EP2002/011200
(87) Internationale Veröffentlichungsnummer: WO 2003/035884

(56) Entgegenhaltungen:
- EP-A- 0 841 396
- WO-A-00/61617
- WO-A-98/32866
- WO-A-99/01560
- DE-A- 19 933 089

## Beschreibung

Die vorliegende Erfindung beschäftigt sich mit Verfahren zur Gewinnung von Zellen, die z.B. in ein Organ transplantierbar sind. Allgemein betrifft die vorliegende Erfindung degenerative Erkrankungen, denen der Untergang definierter Zellpopulationen gemeinsam ist, sowie Transplantate und Arzneimittel zur Behandlung derartiger degenerativer Erkrankungen.

In den Industrieländern gibt es insbesondere durch die sich verändernde Alterspyramide eine Zunahme chronisch degenerativer Erkrankungen, die schwer oder noch gar nicht therapierbar sind. Zu diesen Erkrankungen zählen unter anderem Herzmuskelerkrankungen, neurodegenerative Erkrankungen, Knochen- und Lebererkrankungen, die sich durch den Verlust von relevanten Zellpopulationen auszeichnen.

Beim Herzinfarkt z.B. gehen Herzmuskelzellen irreversibel zugrunde, beim Insulin-abhängigen Diabetis Mellitus gehen die Inselzellen des Pankreas infolge einer Autoimmunerkrankung zugrunde, bei Parkinson gehen die Dopamin-produzierenden Zellen in der Substantia Nigra zugrunde, um nur einige der wichtigsten Erkrankungen zu nennen.

Natürliche Regenerationsprozesse sind fast in keinem Fall in der Lage, diese funktionell wichtigen Zellen zu ersetzen. Daher wird ein großer Fortschritt in der Therapie degenerativer Erkrankungen darin gesehen, solche Organ-bezogenen Zellen außerhalb des Körpers zu züchten und nach entsprechender Vermehrung in die geschädigten Organe zu transplantieren. Handelt es sich um körpereigene Zellen, ist eine nachhaltige Regeneration der Organe wahrscheinlich, da keine Gewebeabstoßungsreaktionen stattfinden.

Solche Organ-bezogenen Zellen können heute aus embryonalen und auch aus adulten Stammzellen gewonnen werden. So ist es möglich, Herzmuskelzellen aus mesenchymalen Stromazellen des Knochenmarks zu gewinnen. Diese Zellen können sich aber nur begrenzt teilen und die Anzahl der Zellteilungen reicht nicht aus, um die erforderliche Anzahl von Organ-bezogenen Zellen zu erhalten. Daher wird angestrebt, solche Zellen zu immortalisieren, um diese in unbegrenzter Menge produzieren zu können. Eine Immortalisierung ist dadurch möglich, daß man die Genfunktion für zumindest die katalytische Untereinheit der humanen Telomerase (hTRT) in primäre Zellen einbringt. In vielen Fällen sind noch weitere Genfunktionen notwendig, um den Zellzyklus-Arrest primärer Zellen zu überwinden, damit diese überhaupt erst anfangen, sich zu teilen. Diese Genfunktionen haben meist transformierende bzw. oncogene Eigenschaften. Ein Prototyp dieser Genfunktionen ist das Large Tumor Antigen von SV40.

Seit langer Zeit ist bekannt, daß primäre Zellkulturen eine begrenzte Zellteilungsfähigkeit haben. Leonard Hayflick vom Wistar Institut entdeckte 1961, daß Fibroblasten von Neugeborenen 80-90 Zellteilungen machen können, die von 70-jährigen Personen teilten sich nur noch 20-30 mal. Nach diesen Teilungszahlen gehen die Zellen in die Seneszenz. Die replikative Kapazität ist durch das Spenderalter festgelegt.

Heute ist bekannt, daß diese replikative Kapazität durch die Länge der Telomere, das sind die Chromosomenenden, determiniert ist. In normalen Zellen verkürzen sich die Telomere bei jeder Zellteilung. Die Telomere bestehen aus Wiederholungen einer Hexamer-Sequenz, TTAGGG bei Säugern, und sind beim neugeborenen Menschen ungefähr 12 kb lang. Dieser Verlust findet in den meisten somatischen Zellen statt. Zellen der Keimbahn haben eine Enzymfunktion, die diesen Replikationsverlust wieder ausgleichen kann. Diese sogenannte Telomerase wurde zuerst beim Einzeller Tetrahymena, einem Ciliaten, von Elizabeth Blackburn und Carol Creider entdeckt. Es handelt sich um ein Ribonukleoprotein. Der RNA-Anteil, von einem separaten Gen kodiert, enthält die Template-Sequenz für die Telomerase-Reaktion. Das Gen für diese Template-RNA konnte inzwischen aus vielen Organismen inklusive Mensch kloniert werden. Inzwischen wurden auch die anderen Faktoren der Telomerase aus verschiedenen Spezies kloniert. Die Telomerase besteht zusätzlich noch aus einem P80-Protein, welches das RNA-Template bindet, und einem P95-Protein, welches die Polymerase-Funktion stellt. Es handelt sich bei der Telomerase also um eine spezielle reverse Transkriptase, die anhand einer gebundenen RNA ein Stück DNA an den Chromosomenenden erzeugt.

Durch Telömer-bindende Proteine wird dabei sichergestellt, daß die Verlängerung der Chromosomenenden reguliert erfolgt. Aus menschlichen Zellen wurde das Gen für den 60 kDa großen Telomer-Repeat-Faktor TRF kloniert. Das Protein hat eine DNA-bindende Domäne, die Homologie zum MYB-Onkoprotein aufweist und sich auch im homologen Hefe-Protein RAP1 wiederfinden läßt. Bindung von TRF sowie weiteren Proteinen an das Telomer bewirkt eine besondere Verpackung des Chromosomenendes. Dadurch wird die Telomerase nachweislich gehemmt. Je kürzer das Telomer, desto geringer ist die Hemmung, wodurch eine Telomerhomöostase gewährleistet wird. Diese Homöostase hat aber sehr wahrscheinlich noch eine andere wichtige Funktion: Sie koppelt die Telomerregulation mit dem Zellzykluskontrollsystem. Dieses wird über einen p53-abhängigen Mechanismus aktiviert, wenn DNA-Brüche bzw. nackte DNA-Enden , vorliegen. In alternden, Telomerase-negativen somatischen Zellen kommt es zu einer sukzessiven Erosion der Telomere und damit auch der Bindungsmöglichkeiten von TRF und verwandter Proteine. Man hat experimentellen Anhalt dafür, daß bei Unterschreitung einer Mindestlänge das p53-abhängige Checkpoint-System aktiviert wird, so daß der Zellzyklus am Übergang G1/S angehalten wird. Die Zelle ist am sogenannten Hayflick-Seneszenz-Limit angekommen.

Durch Infektionen von Zellen mit Krebs-auslösenden Viren kann dieser Punkt überschritten werden. Ein solches Virus ist bspw. das SV40. Dieses Virus exprimiert das sogenannte Large Tumor Antigen, TAg, welches an die Tumorsuppressorproteine p53 und pRB bindet und diese dadurch inaktiviert. Dies führt zu einem Defekt des Checkpoint-Systems. Damit ist es für eine Zelle möglich, sich über das Hayflick-Limit hinaus zu teilen. Sie hat eine verlängerte Lebensspanne (extended life span). Die entstehende Zellpopulation ist aber noch nicht unsterblich, also noch nicht immortalisiert, da es noch einen zweiten Kontrollpunkt gibt: Dieser wird Krise genannt und entsteht durch weiteren Verbrauch der Telomere. Ab einer Telomer-Länge von ungefähr 2,5 kb wird das Chromosomenende instabil. Möglicherweise ist daran auch der zelluläre Rekombinationsapparat beteiligt. Die genetische Instabilität ist für die allermeisten Zellen letal. In ganz seltenen Fällen, weniger als 1 pro 10 Millionen, entgeht eine Zelle dieser Krise und tritt wieder in das replikative Leben ein. Eine solche Zelle ist immortalisiert und damit eine potentielle Krebszelle.

Immortalisierte Zellen und Tumorzellen haben in mehr als 90 % der Fälle eine Expression der katalytischen Untereinheit der Telomerase. Diese ist limitierend, während die Template RNA und das TP1 in den meisten Zellen exprimiert zu sein scheint. Die meisten somatischen Zellen sind dagegen negativ für die katalytische Untereinheit der Telomerase. Ausnahmen von dieser Regel sind aktivierte T- und B-Lymphozyten, CD34 positive Stammzellen, sowie mitotisch aktive Keratinozyten. Man hat aber gefunden, daß die meßbare Telomeraseaktivität in diesen Zellen bestenfalls den Telomerverlust verlangsamen kann, stoppen kann sie ihn nicht. Andererseits hat man auch einige humane Tumore ohne Telomerase-Aktivität gefunden. Da diese Tumoren oft besonders lange Telomere aufweisen, vermutet man, daß es alternative Mechnismen gibt, um den Telomerverlust auszugleichen.

Um Zellen, die sich bereits teilen, zu immortalisieren (z.B. primäre Fibroblasten), ist die Zugabe der katalytischen Untereinheit der Telomerase ausreichend. Ruhende und terminal differenzierte Zellen (z.B. adulte Herzmuskelzellen, Neuronen) benötigen zusätzlich noch Genfunktionen, um den Zellzyklus-Arrest zu überwinden. Dazu können virale Oncogene wie SV40 TAg, HPV E6 und E7, Adenovirus E1A und E1B verwendet werden. Aber auch zelluläre Oncogene wie ras, myc, src etc. können die notwendigen Wachstumssignale bereitstellen.

Das inhärente Problem jeder Immortalisierung ist aber, daß solche Zellen sich durch Anhäufung von Mutationen zu Krebszellen weiterentwickeln können. Daher ist es notwendig, die Immortalisierung wieder rückgängig machen zu können.

In der nicht vorveröffentlichten DE 100 19 195 wird zur Lösung dieses Problems eine reversible Immortalisierung vorgeschlagen, die auf der Einschleusung eines "Survive-Genkomplexes" in organbezogene Zellen beruht. Dieser Genkomplex enthält u.a. die katalytische Untereinheit der humanen Telomerase sowie das TAg. Der Komplex ist flankiert durch Lox/P Sequenzen. Die Zellen werden durch die immortalisierende Eigenschaft des Komplexes ex vivo beliebig lange vermehrt. Vor der Transplantation in Patienten wird die Cre-Rekombinase angewandt, welche den Survive-Komplex zwischen den Lox/P Sequenzen herausschneidet. Diese Technik erfordert für den Einsatz am Menschen, daß es gewährleistet ist, daß die Immortalisierungsfunktionen aus jeder Zelle vollständig entfernt werden.

Gemäß der DE 100 19 195 erfolgt dies durch die Kombination des Cre/Lox Systems mit dem negativen Selektionssystem der HSV-Thymidinkinase (TK). Alle Zellen, in denen der Survive-Genkomplex noch funktionell ist, werden durch die Aktivität der TK abgetötet, wenn das Prodrug Ganciclovir auf die Zellen gegeben wird. Ein Nachteil dieser Technologie kann darin gesehen werden, daß der Survive-Genkomplex in Form einer exprimierbaren DNA-Sequenz verabreicht wird, die random ins Genom integrieren kann. Selbst nach erfolgreicher Anwendung der Cre-Recombinase ist daher nicht auszuschließen, daß die zu den LoxP-Stellen distalen DNA- Sequenz im Genom verbleiben.

Ferner beschreibt WO 00 61617 A Fusionsproteine auf der Basis von VP22 oder TAT, die mit Transformationsproteinen fusioniert sind und zur Immortalisierung verwendet werden können.

DE 199 33 089 A beschreibt ein Fusionsprotein bestehend ausschließlich aus VP22 und dem SV40 Tag, daß zur Zell- und Geweberegeneration geeignet sein soll.

WO 00/31238 A beschreibt Verfahren zur Erhöhung der proliferativen Kapazität und zum Verhindern der Seneszenz von Zellen. Hierzu werden chimäre Proteine angestrengt, ohne jedoch diese weiter zu offenbaren.

EP 841396 A offenbart Fusionsproteine die hTRT enthalten, jedoch nicht die transiente Immortalisierung betreffen.

Vor diesem Hintergrund liegt der vorliegenden Erfindung die Aufgabe zugrunde, ein Verfahren, mit dem sowohl die Immortalisierung von Zellen für die Erzeugung regenerativen Gewebes als auch die vollständige Remortalisierung der Zellen möglich ist, sowie geeignete Mittel zur Verwendung in dem neuen Verfahren bereitzustellen.

Erfindungsgemäß wird diese Aufgabe gelöst durch ein Verfahren zur transienten Immortalisierung von Zellen, bei dem von aussen Immortalisierungsproteine mittels Nanopartikel oder Liposomen in die Zellen eingebracht werden.

Unter Immortalisierungsproteinen werden im Rahmen der vorliegenden Erfindung einerseits Transformationsproteine verstanden, die bei ihrer Expression in der Zelle dafür sorgen, dass die entsprechende Zelle sich wieder bzw. über das Hayflick- Limit hinaus weiter teilt, wie es bspw. das TAg des SV40 bewirkt.

Die Applikation eines solchen Immortalisierungsproteins sorgt dafür, dass z. B. eine ruhende, terminal differenzierte Zelle sich wieder teilt, so dass aus Ausgangszellen eines Organs ex vivo Gewebe für eine Transplantation produziert werden kann.

Andererseits werden unter Immortalisierungsproteinen im Rahmen der vorliegenden Erfindung auch Telomerproteine verstanden, die bei ihrer Expression in der Zelle dafür sorgen, dass die entsprechende Zelle unbeschränkt replikationsfähig bleibt oder wieder wird, da der Telomerverlust bei der Expansion vermieden wird, wie es bspw. die katalytische Untereinheit der Telomerase bewirkt. Die Anmelderin hat ein Plasmid, das ebenfalls Gegenstand der vorliegenden Erfindung ist und für einehTRTP1us genannte katalytische Untereinheit der humanen Telomerase kodiert, am 17.10.2001 bei der DSMZ (DSM 14569) nach dem Budapester Vertrag hinterlegt, es trägt die Bezeichnung pcrscript-Telomerase und ist in E.coli HB101 transfiziert. Aus dem Plasmid läßt sich die DNA-Sequenz für das Immortalisierungsgen hTRT^{plus} isolieren.

Die Transformations- und Telomerproteine werden nun erfindungsgemäß für sich oder in Kombination solange zu zu expandierenden Zellen zugegeben, bis die gewünschte Menge an Gewebe produziert wurde.

Die Transformations- und Telomerproteine lassen sich aber auch mit einem der unten noch zu beschreibenden Verfahren und in der noch weiter zu beschreibenden Ausgestaltung für eine Applikation in Patienten einsetzen, um eine transiente Anregung der Zellteilung in vivo zu erzielen (transiente In-vivo-Immortalisierung).

Ein wesentlicher Vorteil des neuen Verfahrens ist darin zu sehen, daß keine DNA-Sequenzen in die Zellen übertragen werden, so daß eine Integration ins zelluläre Genom nicht stattfinden kann. Die "Immortalisierung" hält nur so lange an, wie die Immortalisierungsproteine fortlaufend von außen appliziert werden. Absetzen der Zufuhr von Immortalisierungsproteinen führt zur Reversion der Immortalisierung, da die in den Zellen befindlichen Immortalisierungsproteine durch zelleigene Proteasen laufend abgebaut werden. Dieser Vorgang wird im Rahmen der vorliegenden Erfindung als transiente Immortalisierung bezeichnet, da sie nur solange anhält, wie die Immortalisierungsproteine extern zur Verfügung gestellt werden.

Diese Proteine können dafür z.B. von Feederzellen sezerniert oder rekombinant mit z.B. dem Baculovirussystem oder in E.coli produziert werden.

Die Genfunktionen mit Immortalisierungseigenschaften wirken also nicht als exprimierbare DNA-Sequenz, sondern unmittelbar als. Proteine auf die zu immortalisierenden Zellen. Dazu werden die zu immortalisierenden Zellen mit Immortalisierungsproteinen versetzt, die mittels biochemischer, chemischer oder physikalischer Applikation in die Zellen übertragen werden.

Bei einer biochemischen Applikation der Immortalisierungproteine werde diese mit Proteintransduktions-Domänen, Liganden, z.B. Peptid-Liganden, oder Single Chain Antikörpern fusioniert. Die Immortalisierungsproteine werden dazu entweder rekombinant hergestellt, z.B. im Baculovirus-System oder E.coli-System, und als gereinigte Fusionsproteine direkt auf die Zielzellen, also die organbezogenen Zellen gegeben, oder in Feederzellen exprimiert, die die Immortalisierungsproteine ins Medium abgegeben. Die Feederzellen werden mit den Zielzellen kokultiviert, so daß die Immortalisierungsproteine aus den Feederzellen ins Medium gelangen und von den organbezogenen Zellen aufgenommen werden. Die Feederzellen können dabei verschiedene Fusionsproteine exprimieren, wobei es aber auch möglich ist, daß verschiedene Feederzellen eingesetzt werden, von denen jede Art nur ein Fusionsprotein exprimiert.

Eine chemische Applikation der Immortalisierungsproteine erfolgt bspw. über Liposomen oder internalisierbare Nanopartikel. Die Immortalisierungsproteine werden rekombinant hergestellt, gereinigt und mit diesen chemischen Verfahren in die Zielzellen eingebracht. Ebenso können die Immortalisierungsproteine an einen Nicht-Peptidliganden chemisch gekoppelt und über diesen in die Zielzellen eingeschleust werden.

Eine physikalische Applikation der Immortalisierungsproteine erfolgt durch Partikelbeschuß, Elektroporation oder Mikroinjektion. Die Immortalisierungsproteine werden rekombinant hergestellt, gereinigt und mit diesen physikalischen Verfahren in die Zielzellen eingebracht.

Für die biochemische Applikation werden die Immortalisierungsproteine mit zusätzlichen Aminosäuren am Amino- oder Carboxyterminus versehen, die eine Aufnahme der Proteine aus dem Zellkulturmedium über natürliche Transportvorgänge gestatten. Dies kann durch Erzeugung von Fusionen aus Immortalisierungsproteinen mit Proteintransduktionsdomänen geschehen. Proteine mit solchen Domänen werden als "Messenger" oder "Translocating" Proteine bezeichnet (Übersicht in: Prochiantz, Curr. Opin. Cell Biol. 2000, 12. 400-406).

Viele der heute bekannten Messenger Proteine gehören zu den Homeoproteinen (z.B. Engrailed, Hoxa-5, Antennapedia). Homeoproteine sind Transkriptionsfaktoren, die eine wichtige Rolle bei Entwicklungsprozessen spielen und bei allen Metazoen sowie bei Pflanzen vorkommen. Die Transkriptionsfaktoren binden mit einer 60-Aminosäuren großen Domäne, der Homeodomäne, an die DNA. Die Homeodomänen enthalten drei Helices. Bei dem Homeoprotein Antennapedia wurde entdeckt, daß die Aminosäuren 43-58 in der dritten Helix die "Cellular Import Sequence", CIS, darstellen. Aus dieser Sequenz wurde die Familie der Penetratin-Peptide entwickelt (Übersichtsartikel in: Derossi et al., Trends Cell Biol 1998, 8: 84-87), wobei Penetratin-1 die Originalsequenz ist.

Sowohl das gereinigte Pentratin-1 als auch Fusionen von Pentratin-1 mit heterologen Proteinen oder Peptiden werden über einen untypischen Vorgang, der nicht den Endozytoseweg umfasst, direkt vom extrazellulären Raum in das Cytoplasma oder den Nukleus aufgenommen. Der genaue Mechanismus ist noch nicht verstanden. Die Firma Q-BIOgene (Heidelberg) bietet zwei Möglichkeiten der Anwendung von Penetratin an: 1. Penetratin-1-Peptid wird chemisch an die zu importierenden Proteine oder Peptide gekoppelt; diese Fusionsproteine werden dann auf die Zellen gegeben und von diesen aufgenommen. 2. Mit dem TransVector System von Q-BIOgene wird die DNA-Sequenz des Zielproteins mit der DNA-Sequenz des Pentratins fusioniert; das Fusionsprotein kann dann nach Transformation des Vectors in E.coli Bakterien rekombinant hergestellt und über ein HIS-Tag gereinigt werden. Das rekombinante Fusionsprotein wird auf die Zielzellen gegeben und von diesen aufgenommen.

Die Firma Q-BIOgene Heidelberg beschreibt, daß Penetratin 1 erfolgreich mit Proteinen angewendet werden kann, die über 100 Aminosäuren groß sein können. Daher ist die Verwendung von Penetratin oder davon abgeleiteten Peptide für den Transport der Telomerase oder des T-Ag auch Gegenstand der Erfindung.

Eine im Rahmen der Erfindung vorgesehene Applikationsweise ist die Fusion der Immortalisierungsproteine mit dem Voyager Protein VP22. Dieses 38 kDa große Protein ist das Genprodukt des Herpes simplex Virus (HSV) Gens UL49 und ein Hauptstrukturprotein des HS-Virions. Es zeigt die besondere Eigenschaft des interzellulären Transports, d.h. es wird aus der Zelle, in der es synthetisiert wurde, heraus und in den Kernbereich der benachbarten Zellen transportiert, wie in der Literatur beschrieben (Elliot and O'Hare, Cell 1997, 88: 223-233).

Interessanterweise behalten auch Fusionsprotein von VP22 z. B. Fusionsproteine VP22-GFP (Green fluorescent protein) diese Eigenschaft bei.

Von den Erfindern der vorliegenden Anmeldung wurde eine Fusion von VP22 mit dem Large T Ag von SV40 durchgeführt sowie eine Zelllinie erzeugt, die dieses Fusionsprotein bildet und sezerniert. Die Erfinder konnten erstmals nachweisen, dass Fusionen von Proteinen mit VP22 einen Transport der Zielproteine nicht nur in Zellinien, sondern auch in primäre Zellen gestattet.

Zur Durchführung der Erfindung fällt auch die Erzeugung von Fusionsproteinen aus VP22 und Telomerase sowie die Herstellung von entsprechenden Feederzellinien. Die Feederzellen werden zusammen mit den zu immortalisierenden Zellen kultiviert. Die Immortalisierungsproteine werden abgegeben und von den zu immortalisierenden Zellen aufgenommen. Die Feederzellen sind räumlich durch eine Kammer mit einer semipermeablen Membran von den Zielzellen separiert. Durch Herausnehmen der Kammer aus der Zellkulturschale unterbleibt die Versorgung mit den Immortalisierungsproteinen, die Zielzellen sind wieder mortal und im ursprünglichen Zustand.

Neben VP22 und Pentratin gibt es eine Reihe weiterer Proteine bzw. Peptide mit der Fähigkeit, in Zielzellen einzudringen (siehe Tabelle 1). Die Immortalisierungsproteine können mit einem oder mehreren dieser Proteine bzw. Sequenzen aus diesen Proteinen fusioniert werden, ohne dass der Rahmen der Erfindung verlassen wird. Es versteht sich außerdem, daß auch nicht in Tab.1 aufgeführte sowie heute noch nicht bekannte Proteintransduktionssequenzen mit den Immortalisierungsproteinen fusioniert werden können.

**Tab. 1: Messenger Proteine**

| Peptide/Protein | Herkunft | Lokalisation in der Zelle |
|---|---|---|
| FGF-1 und FGF-2 | u.a. Mensch | Nucleus |
| Lactoferrin | u.a. Mensch | Nucleus |
| VP22 | Herpes Simplex Virus | Nucleus |
| TAT | humanes Immundefizienz Virus | Nucleus |
| Engrailed | u.a. Mensch | Nucleus |
| Hoxa-5 | u.a. Mensch | Nucleus |
| Antennapedia homeodomain Peptide ("Penetratin") | Drosophila | Nucleus |

Eine weitere im Rahmen der Erfindung vorgesehene Möglichkeit von Immortalisierungsproteinen ist die Fusion dieser Proteine mit einem Rezeptor-Liganden oder mit einem rekombinanten Single Chain Antikörper, der an einen Rezeptor binden kann. Ein Prototyp für einen universell einsetzbaren Liganden ist das RGD-Motiv, das in Adhäsionsmolekülen wie Vitronectin, Collagen und Laminin vorkommt sowie in Kapsidproteinen vieler Viren wie Coxsackievirus A9 und Adenovirus. Das RGD-Motiv enthält die Aminosäuren Arg-Gly-Asp und vermittelt die Bindung an Integrine, das sind heterodimere Membran-Gylcoproteine, die von fast allen Zelltypen exprimiert werden. Viren können über diesen Mechanismus in Zellen eindringen. Hart beschreibt in Exp. Nephrol. 1999, 2:193-199 die Nutzung von RGD-Liganden für den Transfer von Molekülen in Zellen. Im Rahmen der Erfindung wird das RGD-Motiv an die Immortalisierungsproteine Telomerase und TAg fusioniert. Die Anwendung kann im Rahmen einer Sekretion der Fusionsproteine von kokultivierten Feederzellen stattfinden oder als rekombinant im Baculovirus- oder E.coli System hergestellte Fusionsproteinen.

Es versteht sich, dass auch andere Liganden inkl. Single Chain Antikörper an die Immortalisierungsproteine fusioniert oder (chemisch) gekoppelt werden können, ohne dass der Rahmen der Erfindung verlassen wird. Zur Identifikation von Peptid-Liganden über Phage Display findet sich ein Ausführungsbeispiel in der Beschreibung.

Im Rahmen dieser Erfindung ist die Anwendung von bispezifischen Antikörpern mit Immortalisierungsproteinen vorgesehen. Arndt et al. (Blood 1999,94 : 2562- 2568) beschreiben die Nutzung eines rekombinanten bispezifischen monoklonalen Antikörpers, welcher auf der einen Seite an das CD16-Antigen von Natural-Killer-Zellen bindet und auf der anderen Seite das CD30-Antigen von humanen Hodkin Tumoren erkennt. Die Anwendung des "Diabody" verursacht die Lyse der Tumorzellen durch die Natural Killer Zellen. Die Firma Affimed Therpeutics AG, Heidelberg, bietet als Service-Leistung die Entwicklung spezieller bispezfischer Antikörper an. Im Rahmen der Erfindung können solche bispezifischen Antikörper eingesetzt werden, die auf der einen Seite das zuvor rekombinant hergestellte Immortalisierungsprotein Telomerase oder TAg binden, auf der anderen Seite an einen zellulären Rezeptor binden und dadurch die Internalisation der Immortalisierungsproteine bewirken.

Die chemische Applikation nutzt z. B. Kationische Lipide, die seit längerer Zeit eingesetzt werden, um Nukleinsäuren (Plasmide, Vektoren, Ribozyme etc. ) über die Ausbildung von Liposomen in Zellen einzuschleusen. Zelphati et al. (J. Biol. Chem. 2001,37 : 35103-35110) beschreiben erstmals die Anwendung des neuen trifluoroacetylierten Lipopolyamins TFA-DODAPL zusammen mit dem Dioleoyl Phosphatidylethanolamin DOPE. Mit dieser kationischen Formulierung, die unter dem Handelsnahmen BioPorter von der Firma Gene Therapy Systems Inc. (10190 Telesis Court, San Diego, CA 92121, USA) vertrieben wird, ist es möglich, Peptide und Proteine mit einer hohen Effizienz in Zellen einzuschleusen. Im Rahmen der Erfindung kann das BioPorter-Reagenz verwendet werden, um die zuvor rekombinant hergestellten immortalisierungsproteine Telomerase und SV40 T-Ag in die zu immortalisierenden primären Zellen einzuschleusen. Es versteht sich, dass auch andere geeignete liposomale-Reagentien verwendet werden können, ohne den Rahmen der Erfindung zu verlassen.

Erfindungsgemäß werden Nanopartikel oder Liposomen als Carrier für Immortalisierungsproteine, insbesondere nach einer der vorstehenden Ausführungsformen, verwendet. Diese Nanopartikel oder Liposomen haben zudem den Vorteil eines wesentlich stabileren Einschlusses der Substanzen. Soppimath et al. (Journal of Controlled Release 2001, 70 : 1-20) beschreiben die Herstellung und Verwendung biodegradierbarer Nanopartikel aus Poly (D, L-Lactide) (PLA), Poly (D, L-Glycolide) (PLG), Poly (Lactide-co-Glycolide) (PLGA), Poly (Cyanoacrylate) (PCA) und Poly (e-Caprolactone) (PCL). Nanopartikel haben eine Grösse von 10-1000 nm und können verwendet werden, um DNA, RNA sowie Proteine/Peptide zu verpacken.

Erfindungsgemäß werden internalisierbare Nanopartikel verwendet, die in der Zelle die zuvor rekombinant hergestellten Immortalisierungsproteine Telomerase und T-Ag sukzessive freigeben. Es kann für spezielle Anwendungen von Bedeutung sein, die Oberfläche der Nanopartikel so zu modifizierten, dass eine Bindung an internalisierbare Rezeptoren erreicht wird. Dies kann z. B. durch kovalente oder nicht kovalente Bindung von Liganden oder rekombinanten Single Chain Antikörpern (mono-oder bispezifisch) an die Oberfläche der Nanopartikel erreicht werden.

Auch andere Modifikationen zur besseren Anlagerung der Nanopartikel an Zellen und Aufnahme in Zellen sind möglich, ohne daß der Rahmen der Erfindung verlassen wird. Zur besseren Aufnahme der Nanopartikel in Zellen kann auch eine Elektroporation durch geführt werden.

Biodegradierbare Nanopartikel mit darin verpackten Immortalisierungsproteinen sind besonders dazu geeignet, im Rahmen einer Therapie oder Prophylaxe einem Patienten auch in vivo appliziert zu werden, um eine in-vivo-Regeneration z.B. des Herzen herbeizuführen.

Die vorliegende Erfindung betrifft ferner ein Verfahren zur Gewinnung von Zellen, mit den Schritten: Bereitstellen von organbezogenen Zellen, transientes Immortalisieren der organbezogenen Zellen durch externe Zufuhr von erfindungsgemäß verwendeten Immortalisierungsproteinen mittels Nanopartikel oder Liposomen, Expansion der immortalisierten Zellen und Remortalisierung der expandierten Zellen durch Beenden der Zufuhr von Immortalisierungsproteinen. Als organbezogene Zellen können dabei multipotente Stammzellen, vorzugsweise mesenchymale Stromazellen oder aber ruhende, terminale differenzierte Ausgangszellen des Organs, vorzugsweise Herzmuskelzellen verwendet werden.

Durch die erfindungsgemäße transiente Immortalisierung sind die so hergestellten Zellen klinisch unbedenklich. Die Zellen können außerdem in unbegrenzter Zahl hergestellt werden.

Wenn multipotente Stammzellen als organbezogene Zellen verwendet werden, erfolgt die Expansion der transient immortalisierten Stammzellen erst nach Zugabe zumindest einer Differenzierungssubstanz, die eine Differenzierung der Stammzellen zu organspezifischen Zellen fördert. Diese differenzierten Zellen werden dann mit dem erfindungsgemäßen Verfahren transient immortalisiert.

Werden dagegen terminal differenzierte Ausgangszellen eingesetzt, so werden diese im Zusammenhang mit der transienten Transformation vorzugsweise auch immortalisiert, so daß sie sich nahezu unbegrenzt expandieren lassen.

Werden zwar differenzierte, aber noch teilungsfähige Zellen eingesetzt, wie z.B. neonatale Herzmuskelzellen, so ist keine Transformation sondern nur eine Immortalisierung mit Telomerproteinen vorgesehen.

Mit den so hergestellten Zellen kann z.B. eine Transplantation in einem Herzinfarktareal durchgeführt werden, wodurch gleichzeitig das Risiko eines kongestiven Herzversagens sowie eines sekundären tödlichen Herzinfarktes erheblich vermindert wird. Das Verfahren eignet sich auch zur Gewinnung regenerativer Knochen- und Knorpelzellen, die bei Knochen- und Knorpeltraumata sowie bei chronischer Knochendegeneration (Osteoporose) eingesetzt werden können. Mit dem Verfahren können auch Leberparenchymzellen für die Leberregeneration sowie dopaminerge Zellen zur Behandlung des Morbus Parkinson hergestellt werden.

Das erfindungsgemäße Verfahren ermöglicht die Produktion beliebiger Mengen primärer Zellen für die extrakorporale Herstellung von Gewebe. Nach dem neuen Verfahren produzierte Endothelzellen oder Glatte Muskelzellen können auf einer Matrix, vorzugsweise einer Biomatrix beispielsweise aus Collagen oder Fibronektin angesiedelt werden, um Herz- oder Venenklappen zu erzeugen.

Bei der Herstellung von z.B. Muskelzellen, vorzugsweise von Herzmuskelzellen, sowie von Knochenzellen, ist es bevorzugt, wenn vor der Expansion der transient immortalisierten Stammzellen eine Differenzierungssubstanz zugegeben wird, die ausgewählt ist aus der Gruppe: Dexamethason, 5'-Azacytidin, Trichstatin A, all-trans-Retinsäure und Amphotericin B. Besonders bevorzugt ist es dabei, wenn zumindest zwei, vorzugsweise vier dieser Differenzierungssubstanzen vor der transienten Immortalisierung verwendet werden.

Obwohl eine Differenzierung von Stammzellen zu Herzmuskelzellen durch Zugabe von 5'-Azacytidin induziert wird, kann die Differenzierung durch Zugabe zumindest einer weiteren Differenzierungssubstanz verbessert werden. Besonders geeignet ist dabei eine Kombination von 5'-Azacytidin und Trichstatin A, was nach Erkenntnis der Erfinder der vorliegenden Anmeldung synergistisch wirkt. Die Differenzierung kann durch die weitere Zugabe von all-trans-Retinsäure und Amphotericin B weiter optimiert werden.

Neben der in der Kombination aus mehreren Differenzierungssubstanzen liegenden synergistischen Wirkung liegt ein weiterer Vorteil darin, daß die nach Erkenntnis der Erfinder vorliegende mutagene Wirkung von 5'-Azacytidin erheblich reduziert oder sogar aufgehoben wird.

Auf diese Weise ist eine unbedenkliche klinische Anwendung der so aus Stammzellen gewonnenen Herzmuskelzellen möglich.

Durch die Zugabe der Differenzierungssubstanz Dexamethason werden die Stammzellen vor der transienten Immortalisierung zu Knochen- und Knorpelzellen differenziert. Auch hier kann ein synergistischer Effekt durch die weitere Zugabe der Differenzierungssubstanzen 5'-Azacytidin, Trichstatin A, all-trans-Retinsäure und Amphotericin B erzielt werden.

Zu der Differenzierungssubstanz Dexamethason sei noch erwähnt, daß Conget und Minguell "Phenotypical and functional properties of human bohne marrow mesenchymal progenitor cells", J.Cell Physiol. Band 181, Seiten 67-73 bereits beschrieben haben, daß sich osteogene Zellen aus mesenchymalen Stromazellen durch Dexamethason-Behandlung gewinnen lassen.

Als organbezogene Zellen können dabei sowohl autologe als auch allogene Zellen eingesetzt werden.

Während der Vorteil der autologen Zellen in der Immuntoleranz liegt, weisen die allogenen Zellen den Vorteil auf, daß sie quasi jederzeit in unbegrenzter Zahl zur Verfügung stehen.

Zur Behandlung eines Patienten können dann folglich zunächst aus allogenen Zellen hergestellte transplantierbare Zellen verwendet werden, während parallel aus autologen Zellen des Patienten weitere transplantierbare Zellen hergestellt werden. Wenn dann genügend autologe transplantierbare Zellen zur Verfügung stehen, werden nur noch diese transplantiert, so daß die Immuntoleranz dann kein Problem mehr darstellt.

Mit Hilfe des neuen Verfahrens und ggf. unter Verwendung der neuen Mittel hergestellte Zellen sind ebenfalls Gegenstand der vorliegenden Erfindung. Diese Zellen können erfindungsgemäß zur Herstellung eines Transplantates für die Regeneration eines Organes oder zur Behandlung chronischer Erkrankungen verwendet werden.

Vor diesem Hintergrund betrifft die vorliegende Erfindung ebenfalls ein Transplantat, das die erfindungsgemäß hergestellten Zellen enthält.

Ferner betrifft die vorliegende Erfindung die Verwendung der Zellen zur Regeneration eines Organs.

Zur Durchführung der Erfindung werden die erfindungsgemäß verwendeten Immortalisierungsproteine sowie für die Immortalisierungsproteine kodierende Nukleinsäuremoleküle und Plasmide zur Expression der Immortalisierungsproteine, sowie zur Expression der Immortalisierungsproteine transformierte Zellen, insbesondere Feederzellen, hergestellt (siehe Beispiele).

Insbesondere können hierzu die am 17.10. 2001 nach dem Budapester Vertrag an der DSMZ in Braunschweig unter den Hinterlegungsnummern DSM 14570 und 14568 hinterlegten Plasmide mit den Bezeichnungen pCMV-VP22-TAg und pcDNA-TAg-VP22, die in E. coli HB101 transfiziert sind, zur Herstellung der Fusionsproteine verwendet werden.

Weitere Vorteile ergeben sich auss der Beschreibung und der beigefügten Zeichnung.

Es versteht sich, daß die vorstehend genannten und die nach-stehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Die Erfindung wird jetzt anhand von Ausführungsbeispielen und der beigefügten Zeichnung erläutert. Es zeigen:
- Fig.1:: Western-Blot Nachweis von TAg im Fusionsprotein;
- Fig.2:: Western-Blot Nachweis von VP22 im Fusionsprotein;
- Fig.3:: Zellanfärbungen, die die Generierung von VP22-TAg exprimierenden Zellinien zeigen;
- Fig.4:: Zellanfärbungen, die den VP22-Import in primäre humane Herzmuskelzellen zeigen;
- Fig.5:: Plasmidkarte pCMV-VP22-TAg;
- Fig.6:: Sequenz des Fusionsgens VP22-TAg aus Fig.5;
- Fig.7:: Plasmidkarte pcDNA-TAg-VP22;
- Fig.8:: Sequenz des Fusionsgens TAg-VP22 aus Fig.7;
- Fig.9:: Plasmidkarte pcrscript-Telomerase; und
- Fig..10:: Sequenz des Telomerasegens hTRT^{plus} aus Fig.9.

### Beispiel 1: Klonierung von Fusionsproteinen aus SV40-TAg und VP22

Es wurden Expressionkonstrukte hergestellt, die eine Expression sowohl von VP22-T-Antigen als auch von T-Antigen-VP22, d.h. sowohl von N- als auch von C-terminalen Fusionsproteinen ermöglichen.

### a. Mutagenese

Hierzu wurde als erstes mittels site-directed PCR Mutagenesis ein Plasmid hergestellt, daß das SV40 T-Antigen ohne Stop-Codon enthält (Primer s. Tab.2). Dieses Plasmid wurde pIND-TAg(-Stop) genannt. Das SV40 TAg wurde von Prof. W. Deppert, Heinrich Pette Institut für Experimentelle Virologie und Immunologie der Universität Hamburg bezogen. Bei der "Site directed" Mutagenesis wurde ein Kit von Stratagene Inc verwendet.

**Tab 2: Primer zur site-directed Mutagenesis**

| Primer |
|---|
| 5'-cctccccctgaacctgaaacaagatctaaatacaattgttgttgttaacg-3' |
| 5'-cgttaacaacaacaattgcattcaggatcttgtttcggttcaaggggagg-3' |

### b. Klonierungen

Aus dem Plasmid pIND-TAg(-Stop) wurde durch einen Doppelverdau mit den Restriktionsendonukleasen EcoRI und BglII ein Stopcodon-freies T-Antigen-Fragment gewonnen.

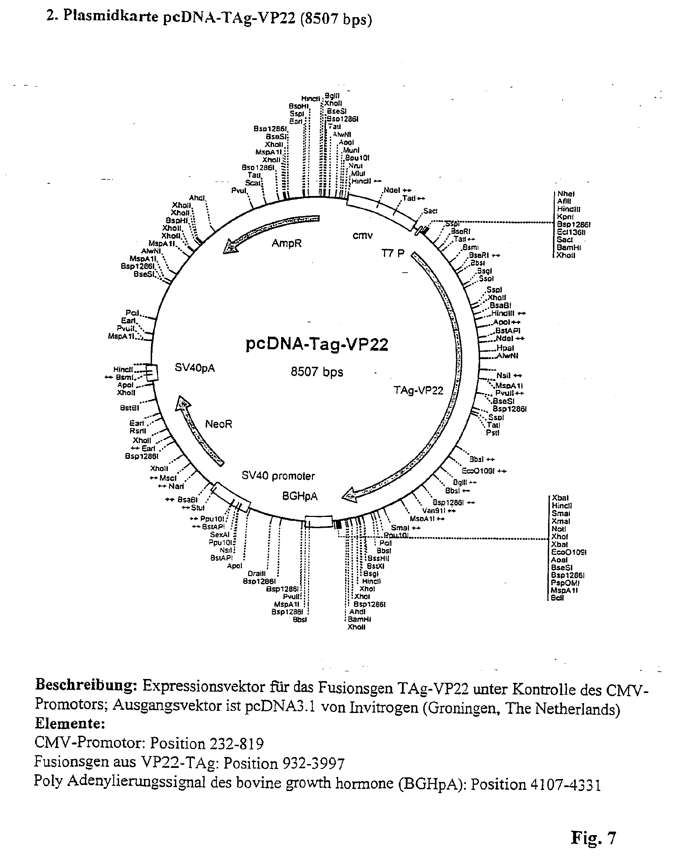

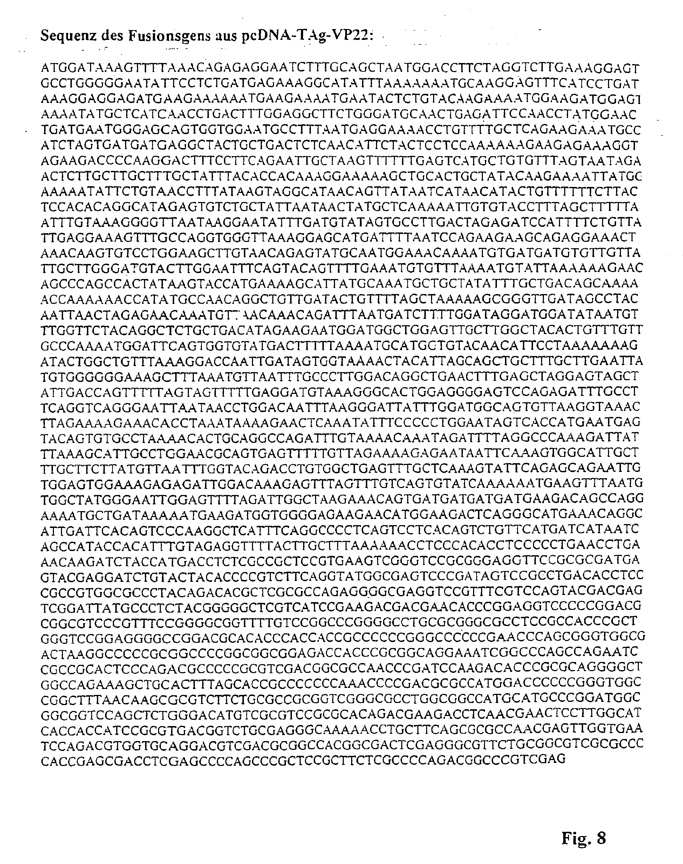

Aus dem Plasmid pCDTK49 wurde das VP22-Fragment mittels NotI und BglII Verdau hergestellt und als Vektor kam pcDNA3.1 (Invitrogen) nach NotI und EcoRI Verdau zur Verwendung. Es ergab sich das Expressionkonstrukt pcDNA-TAg-VP22, welches eine CMV-Promotor gesteuerte Expressionkassette für das Fusionsprotein TAg-VP22 trägt (s. Plasmidkarte und Sequenz von pcDNA-TAg-VP22 in Fig. 7 und 8). Das Plasmid wurde am 17.10.2001 bei der DSMZ nach dem Budapester Vertrag unter der Eingangsnummer DSM 14568 hinterlegt, es ist in E.coli Hub101 transfiziert.

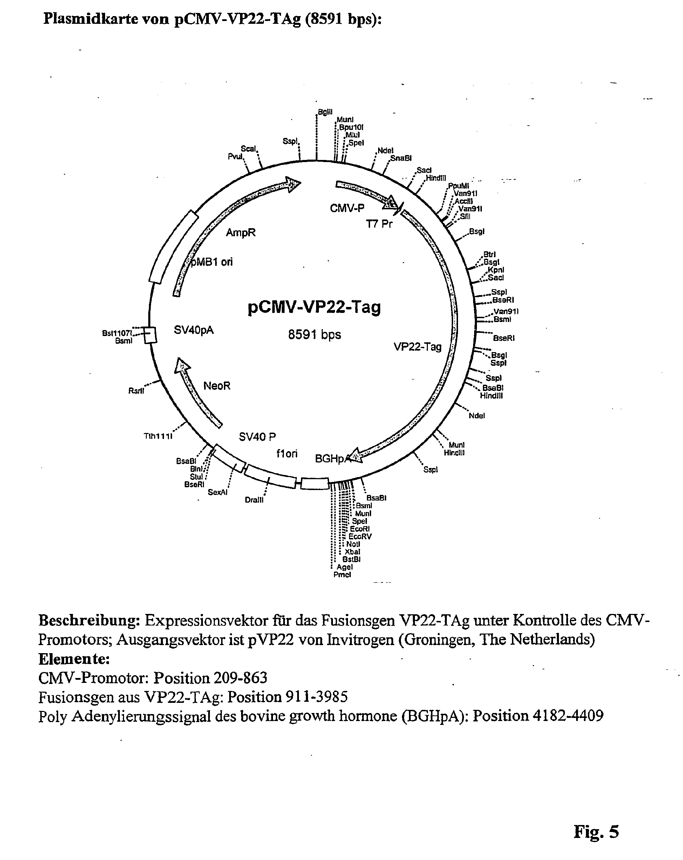

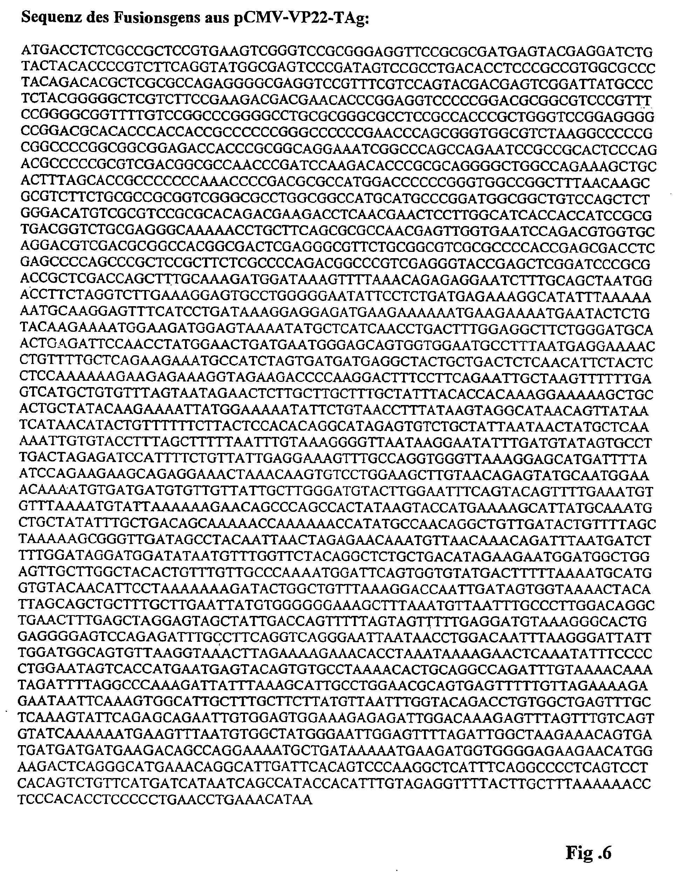

Unter Verwendung des Vektors pVP22 (Invitrogen) wurde das Expressionplasmid pCMV-VP22-TAg hergestellt. Hierzu wurde aus pIND-TAg durch ein Doppelverdau mit KpnI und EcoRI das T-Antigen-Fragment gewonnen und in den ebenfalls mit KpnI und EcoRI geöffneten Vektor pVP22 einligiert. (s. Plasmidkarte und Sequenz von pcCMV-VP22-TAg in Fig. 5 und 6). Das Plasmid wurde am 17.10.2001 bei der DSMZ nach dem Budapester Vertrag unter der Eingangsnummer DSM 14570 hinterlegt, es ist in E.coli HB101 transfiziert.

Das Fusionprotein stellt eine Fusion des VP22-Proteins an den N-Terminus des large T-Antigens dar; die Expressionskassette ist ebenfalls durch den CMV-Promotor gesteuert.

### Beispiel 2: Nachweis des Fusionsproteins aus SV40 TAg und VP22

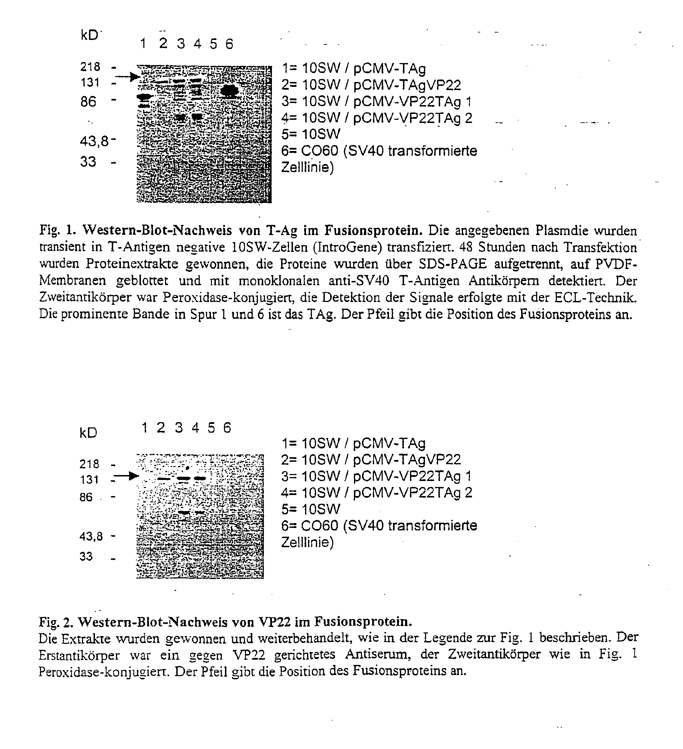

Der Nachweis, daß durch die Klonierungen wirklich Fusionsproteine aus VP22 und SV40 large T-Antigen entstanden sind, wurde in Transfektionsexperimenten mit anschließenden Western Blot Analysen erbracht (Fig. 1 und 2).

Als erstes wurden die neuen Expressionkonstrukte durch transiente Transfektion in T-Antigen negative Zellen eingebracht. Anschließend wurden hieraus Proteinextrakte gewonnen, die in SDS-Polyacrylamidgelen aufgetrennt, auf PVDF-Membranen geblottet und sowohl mit monoklonalen anti-SV40 T-Antigen Antikörpern als auch mit einem polyklonalen anti-VP22 Antiserum analysiert wurden.

Es zeigte sich, daß die mit den Fusionskonstrukten transfizierten Zellen Proteine exprimierten, welche die Größe der zu erwartenden Fusionsproteine zeigten und auch von beiden Sorten Antikörpern erkannt wurden.

### Beispiel 3: Erzeugung von Feeder-Zellinien

Um eine Zelllinie zu generieren, die als "Fusionsproteinproduzent" fungieren kann, wurden 10SW-Zellen (humane, Adenovirus E1A und E1B transformierte Retina-Zellen) mit den Fusionsprotein-Expressionsplasmiden transfiziert und anschließend mit G418 selektiert, da die Expressionskonstrukte auch eine Neomycin-Resistenz vermitteln.

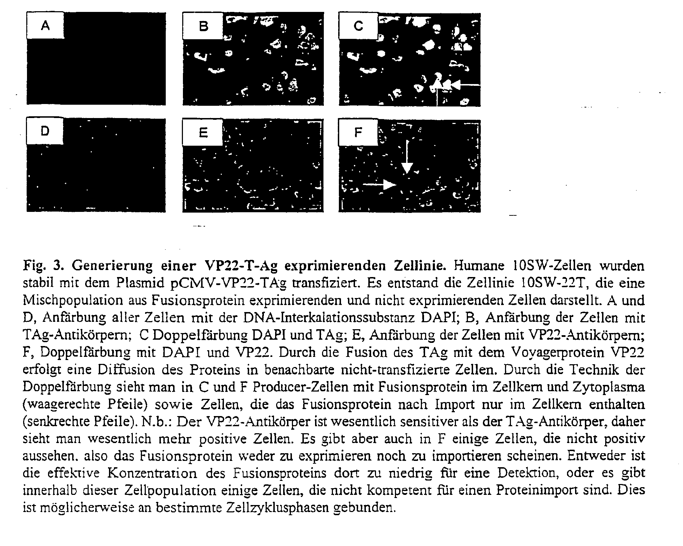

Nach mehrwöchiger Selektion entstand eine Mischpopulation von Zellen, die G418-resistent waren. Immunhistochemische Untersuchungen zeigten, daß - wie zu erwarten war - nicht alle Zellen das Fusionsprotein exprimieren (s. Fig 3). Mittels Insitu-Lokalisationstechniken (Immunfluoreszenz, Doppelanfärbung) konnte die Funktionaliät des Systems nachgewiesen werden. Die meisten Zellen der Feeder-Zellinie produzieren das Fusionsprotein, welches in Nachbarzellen diffundiert. Dies ist daran zu erkennen, daß produzierende und exkretierende Zellen das Fusionsprotein sowohl im Zytoplasma als auch im Zellkern enthalten, während importierende Zellen das Fusionsprotein nur im Zellkern haben (Fig. 3, C und F). Die Zellen werden durch Endpunktverdünnung subkloniert, so daß dann Feeder-Zellinien existieren, welche homogen, d.h. in jeder Zelle das Immortalisierungsprotein exprimieren. Mit diesen Feeder-Zellen konnte der Export des TAg-VP22 Fusionsproteins in primäre Zellen mit Kokultivierungsexperimenten untersucht werden, desgleichen die Funktionalität des TAg in einem NIH3T3-Transformationsassays. Außerdem konnte durch Koimmunpräzipitation die Bindung des TAg an p53 und pRB untersucht werden.

Als Feederzellen können sowohl mortale als auch immortalisierte Zellen verwendet werden.

### Beispiel 4: Import von VP22-Fusionsproteinen in primäre Zellen

Die Erfinder haben folgendermaßen nachgewiesen, daß das VP22-Protein damit fusionierte Proteine auch in primäre Zellen transportiert. Dies war bisher nur für Tumorzellinien bekannt. So wurde untersucht, ob ein VP22-Fusionsprotein-Import auch in humane Fibroblasten, humane glatte Muskelzellen und humane Kardiomyozyten erfolgt.

Hierzu wurden C060-Zellen (eine SV40 transformierte Hamsterzellinie) mit einem rekombinanten Adenovirus infiziert, welches unter Kontrolle des CMV-Promotors ein Fusionsgen aus VP22 und GFP (green fluorescent protein) enthält. Die mit diesem Adenovirus infizierten Zellen wurden gemeinsam mit jeweils einer Sorte der besagten primären Zellen auf sterile Deckgläschen ausgesät. Nach ca. 48-stündiger Inkubation wurden die Deckgläser mit Formaldehyd fixiert und immunhistochemisch untersucht. Hierzu wurde eine Antikörperkombination von Maus-anti-SV40-T-Antigen und Kaninchen-anti-VP22 verwendet.

Die ursprünglich infizierten C060-Zellen konnten also dadurch identifiziert werden, daß sie positiv für das SV40-T-Antigen und für das VP22-GFP-Fusionprotein sind. Primäre Zellen, die das VP22-GFP durch interzelluläre Transportprozesse erhalten haben, sind nur positiv für VP22, nicht aber für das SV40-T-Antigen.

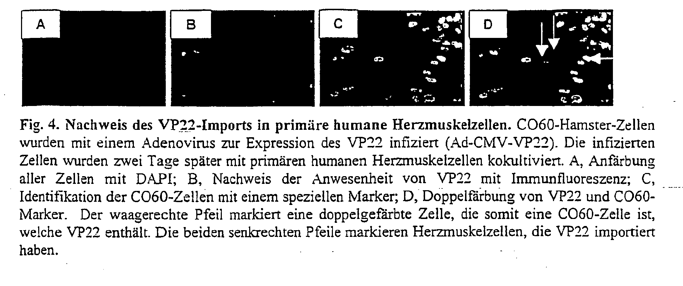

Es zeigte sich; daß ein Transport von VP22-Fusionsproteinen nicht nur in immortalisierte, sondern auch in die untersuchten primären humanen Herzmuskelzellen möglich ist (Fig. 4). Die Funktion des an VP22 anfusionierten Proteins, also des GFP bzw. des Immortalisierungsgens bleibt in den primären Zellen erhalten.

### Beispiel 5. Klonierung der katalytischen Untereinheit der humanen Telomerase

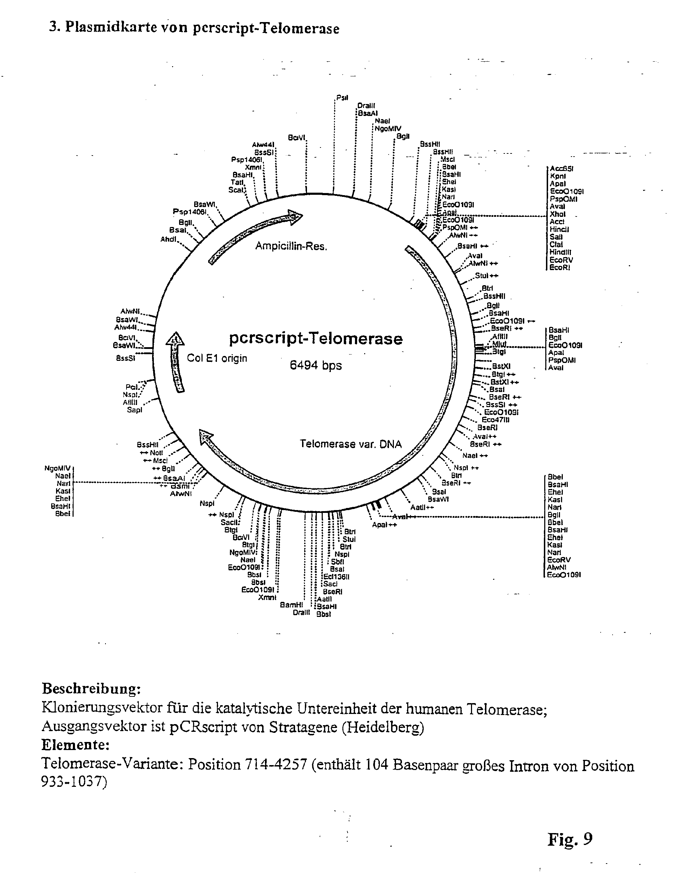

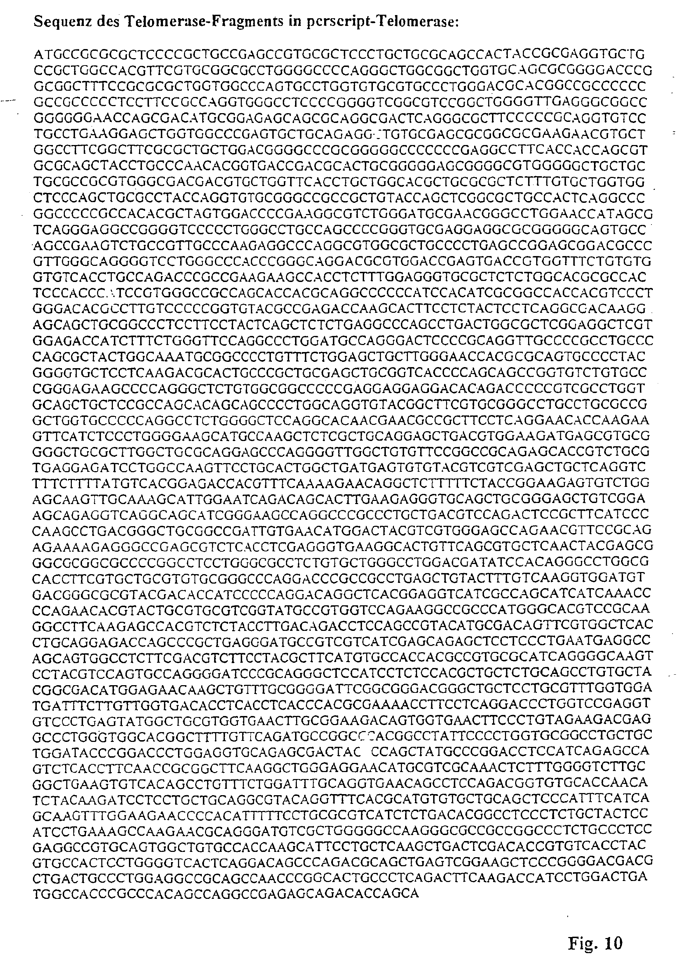

Das für die Immortalisierung wichtigste Gen, die katalytische Untereinheit der humanen Telomerase, wurde aus menschlichen Zellen kloniert (die DNA-Sequenz dieses Telomerase-Fragmentes hTRT^{plus} und die Plasmid-Karte mit der katalytischen Untereinheit von humaner Telomerase finden sich in Fig. 9 und 10). Die cDNA der Telomerase hat einen sehr hohen G/C-Anteil und ist äußerst schwer zu klonieren. Es gibt daher weltweit nur sehr wenige Gruppen, die über eine eigene Telomerase-cDNA verfügen. Die Telomerase aus Fig. 9 und 10 hat im Unterschied zur bekannten und veröffentlichten Telomerase ein Intron von 109 bp. Introns haben einen Transkript-stabilisierenden Effekt. Diese Telomerase wird als Abgrenzung zur bekannten Telomerase als hTRT^{Plus} bezeichnet

Die Telomerase wurde in folgenden Schritten kloniert:
1. Aus humanen Jurkat-Zellen (T-Lymphom-Zelllinie) wurde mRNA gewonnen.
2. Diese wurde mit spezifischen RT-Primern (s. Tabelle 3) in einer reversen Transkription zu cDNA-Molekülen umgeschrieben. (reverse Transkriptase von MMLV).
3. Aus dem so gewonnenen cDNA-Pool wurden in einzelnen PCR-Reaktionen (s. Tabelle 4) Fragmente gewonnen, die jeweils ca. 500 bis 1000bp der kodierenden Bereiche des humanen Telomerasegens umfaßten. Dabei wurden die Primer so gewählt, daß Fragmente entstanden, die sich in solchen Bereichen der Telomerase-cDNA überschnitten, die jeweils eine für diese cDNA einzige Restriktionsschnittstelle enthielten.
4. Das am weitesten 5' gelegene Fragment wurde nicht über reverse Transkription von Telomerase-mRNA generiert, sondern direkt durch PCR genomischer DNA aus Hela-Zellen (humane Zervixkarzinom-Zelllinie) gewonnen. Dabei wurde eine spezielle PCR-Technik für hoch G/C-reiche Sequenzen verwendet.
5. Die erhaltenen Fragmente (Tabelle 5) wurden in Plasmidvektoren einkloniert (Tabelle 6) und in Bakterien vermehrt. Alle Fragmente wurden durch Sequenzierung überprüft. Es wurde ein Basenaustausch an der Position 993 der Telomerase cDNA gefunden. Hier ist das ursprünglich vorhandene C durch ein A ersetzt. Es handelt sich aber um eine stumme Mutation, d.h, dieser Basenaustausch hat keinen Einfluß auf die Aminosäuresequenz.

In verschiedenen 3-Komponenten-Ligationen wurden die einzelnen Fragmente zu einer den ganzen kodierenden Bereich enthaltenden Variante der humanen Telomerase zusammengefügt. Das entsprechende Plasmid heißt pcrscript-Telomerase (Plasmidkarte und Sequenz von hTRT^{plus} in Fig. 9 und 10) und wurde am 17.10.2001 bei der DSMZ nach dem Budapester Vertrag unter der Hinterlegungsnummer DSM 14569 hinterlegt, es ist in E.coli HB 101 transfiziert.

**Tab. 3: Verwendete Primer für die spezifische reverse Transkription von Telomerase mRNA**

| **Primer zur reversen Transkription** | **Sequenz** |
|---|---|
| RT-telo-4 | 5-CTCATATATTCAGTAT-3 |
| RT-telo-3 | 5-CTGGACACTCGCTACA-3 |
| RT-telo-2 | 5-TCAGCCGGACATGCA-3 |
| RT-telo-1 | 5-TCACTCAGGCCTCAG-3 |

**Tab. 4: Verwendete Primer zur Gewinnung von überlappenden PCR-Fragmenten**

| **PCR-Primer** | **Sequenz** | **Restriktionsschnittstelle** |
|---|---|---|
| PCR-telo-10 | 5'-GCTGGTGTCTGCTCTCG-3' | - |
| PCR-telo-11 | 5'-CTGCAGCAGGAGGATCTTGTAGATG-3' | ApaLI |
| PCR-telo-6 | 5'-GCAGGTGAACAGCCTCCAGAC-3' | ApaLI |
| PCR-telo-R13 | 5'-CACAGGCTGCAGAGCAGCGTGGAG-3' | BamHI |
| PCR-telo-F12 | 5'-GTCCTACGTCCAGTGCCAGGGGATC-3' | BamHI |
| PCR-telo-R15 | 5'-GAGCACGCTGAACCAGTGCCTTCAC-3' | XhoI |
| PCR-telo-F14 | 5'-AGAGGGCCGAGCGTCTCACCTCGA-3' | XhoI |
| PCR-telo-R17 | 5'-CGCTCATCTTCCACGTCAGCTCCTGC-3' | SphI |
| PCR-telo-F16 | 5'-CTCAGGAACACCAAGAAGTTCATC-3' | SphI |
| PCR-telo-R19 | 5'-CCTGGCATCCAGGGCCTGGAACCCA-3' | BssSI |
| PCR-telo-F18 | 5'-TCCCTACTCAGCTCTCTGAGGCCCAGC-3' | BssSI |
| PCR-telo-F30 | 5'TTGCTGGTGGCTCCCAGCTGCGCCTAGGA-3' | SexAI |
| PCR-telo-R21 | 5'-AGTGGCAGCGCCGAGCTGGTACAGC-3' | SexAI |
| PCR-telo-7 | 5-ATGCCGCGCGCTCCCCGCTGCCGAG-34 | - |

**Tab. 5: PCR-Fragmente. Fragmente T2 bis T6 wurden durch PCR-Amplifikation von Telomerase cDNA gewonnen.: Fragment T711 wurde durch PCR-Amplifikation genomischer DNA gewonnen und enthält ein Intron.**

| Fragment | Primer | Bereich | Bemerkungen | Restriktionsschnittstellen |
|---|---|---|---|---|
| T2 | F12, 10 | 2524bp- | 3'-Bereich + | 5'-terminal: BamHI |
| | | 3494bp | Stop-Codon | 3'-terminal: - |
| T3 | R13, F14 | 2001 bp- | - | 6'-terminal:XhoI |
| | | 2589bp | | 3'-terminal:BamHI |
| T4 | R15, F16 | 1517bp- | - | 6'-terminal:SphI |
| | | 2051 bp | | 3'-terminal: XhoI |
| T5 | R17, F18 | 1088bp- | - | 5'-terminal:BssSI (BsiI) |
| | | 1596bp | | 3'-terminal:SphI |
| T6 | R19, F20 | 530bp-1179bp - | | 5'-terminal:SexAI |
| | | | | 3'-terminal: BssSI (BsiI) |
| T7II | 7, R19 | 55bp-1176bp | Start-Codon | 5'-terminal:- |
| | | | | 3"-terminal:BssSI (BsiI) |

**Tab. 6: Zwischenkonstrukte mit je einem Fragment**

| **Vektor** | **Insert** |
|---|---|
| pIND-T21 | T2 |
| pIND-T3 | T3 |
| pIND-T4 | T4 |
| pIND-T5 | T5 |
| pIND-T6 | T6 |
| pCRII-T7II | T7II |

### Beispiel 6: Klonierung eines Vektors zur Expression eines Fusionsproteins bestehend aus VP22 und der katalytischen Untereinheit der Telomerase in Säugerzellen

Es wurde ein Vektor konstruiert, welcher die Expression eines Fusionsproteins bestehend aus VP22 und der katalytischen Untereinheit der Telomerase in Säugerzellen erlaubt, wobei die VP22-Sequenzen 5'-wärts der Telomerase-Sequenzen liegen.

### a. Mutagenese des Konstrukts pcrscript-Telomerase

Zur Klonierung des Expressionsvektors wurde zunächst in dem Konstrukt pcrscript-Telomerase ein vor dem Startcodon liegendes Stop-Codon in der Telomerase-Sequenz über 'site-directed PCR mutagenesis' (Kit von Stratagene) entfernt und anstelle dessen eine Kpn I-Schnittstelle eingefügt. Dazu wurde der in Tab.7 angegebene Primer #1 verwendet. Nach PCR mit einem entsprechendem 'reverse' Primer wurde das daraus resultierende Plasmid für eine zweite 'site-directed PCR mutagenesis' verwendet. Diese mit Primer #2 durchgeführte Mutagenese dienten zur Entfernung des am 3' Ende der Telomerase-Sequenz befindlichen Stop-Codons bei gleichzeitiger Einführung einer Age I-Schnittstelle, wodurch eine 'in frame' Fusion mit dem im Vektor pVP22/myc-His (Invitrogen) vorliegenden 'His-tag' möglich wird.

### b. Klonierung des Konstrukts pCMV-VP22-Telo-His

Zur Klonierung des Expressionskonstrukts pCMV-VP22-Telo-His wurde mit den Restriktionsendonuklease Kpn I und Age I ein Fragment aus dem mutagenisierten Plasmid herausgespalten, welches die Telomerase-Sequenzen mit einem am 5' Ende liegenden Start-Codonbeinhaltet, dem jedoch das Stop-Codon am 3' Ende fehlt. Dieses Fragment wurde anschließend gerichtet in den mit Kpn I und Age I geöffneten Vektor pVP22/myc-His der Firma Invitrogen kloniert, so daß ein Fusionsgen bestehend aus N-terminalem VP22, Telomerase und C-terminalem 'His-tag' unter der Kontrolle des CMV-Promotors vorliegt.

**Tab 7: Primer für 'site-directed PCR mutagenesis' zur Generierung des Stop-Codon-freien Telomerase-Fragments. Die Fett markierten Buchstaben geben die Sequenzen der neu inserierten Restriktionsschnittstellen Kpn I (Primer #1) bzw. Age 1 (Primer #2) an.**

| Primer | Sequenz |
|---|---|
| Primer #1 | 5'-aagcttgatatcgaattcgggtaccatgccgcgcgctccccgctcccgg-3' |
| Primer #2 | 5'-gacttcaagaccatcctggacaccggtccacccgcccacagccaggccgag-3' |

### Beispiel 7 : Erzeugung einer Feeder-Zellinie für VP22 Telomerase

Die Herstellung einer VP22-Telomerase-exprimierenden Feeder-Zellinie wurde analog zur TAg-VP22 Feeder-Zellinie durchgeführt, indem das Konstrukt pCMV-VP22-Telo-His in 10SW-Zellen stabil transfiziert wurde. Da das Konstrukt ein Neomycin-Resistenzgen enthält, konnte unter Gabe von G418 auf die das VP22-Telomerase-Protein stabil exprimierenden Zellen selektioniert werden.

### Beispiel 8: Kokultivierung von Feederzellen mit primären Zellen

Die Firma Nunc GmbH, Wiesbaden, liefert spezielle Zell- und Gewebekultureinsätze, die eine Kokultivierung von Feederzellen mit den zu immortalisierenden primären Zellen gestatten.

Die Membranen der Nunc-Einsätze sind für die Anheftung und Proliferation adhärenter Zellen bestimmt. Während der eine Zelltyp (bspw. Feederzellen) auf der Memban kultiviert werden kann, läßt sich ein anderer Zelltyp (bspw. primäre Zellen) im Well-Boden der passenden Multischale halten, ohne daß die beiden Kulturen miteinander in direkten Kontakt treten. Ionen, Proteine und weitere Substanzen können dagegen frei durch die Poren der Membran diffundieren. Durch die Auswahl an verschiedenen Porengrößen kann zudem die Größe der übergetretenen Stoffe festgelegt werden.

Im Rahmen der Erfindung werden die in den vorangegangenen Beispielen beschriebenen Feederzellen auf solche Membraneinsätze ausgesät. Für den industriellen Maßstab wird die Größe der Zellkulturkammern und der dazu passenden Membraneinsätze entsprechend angepaßt.

Es ist auch im Rahmen der Erfindung möglich, Bioreaktoren zur Massenkultivierung sowohl der Feederzellen als auch der primären Zellen zu verwenden. Feederzellen und primäre Zellen werden in zwei separate Kammern innerhalb des Bioreaktors ausgesät. Die Kammern sind durch eine semipermeable Membran getrennt, so daß die Diffusion der Immortalisierungsproteine zu den primären Zellen möglich ist.

Die Feederzellen können auch in einer Mischkultur mit den primären Zellen vorliegen. Für diesen Fall ist es sinnvoll, die Suspensionszellen als Feederzellen zu verwenden und Monolayer Zellen als primäre Zellen oder umgekehrt. Dadurch ist eine mechanische Trennung der Feederzellen von den primären Zellen nach erfolgter Immortalisierung möglich. Die Feederzellen können auch durch die stabile Transfektion eines zytotoxischen Gens (z.B. einer exprimierbaren HSV-Thymidinkinase) selektiv nach Gabe des entsprechenden Prodrugs (in dem Fall Ganciclovir) abgetötet werden.

Auch andere Techniken der Trennung von Feederzellen und primären Zellen sind möglich (z.B. durch Sorting über Oberflächenmoleküle mit einem Fluorescence Activated Cell Sorter [FACS] oder Magnetic Activated Cell Sorter [MACS]).

### Beispiel 9: Klonierung eines baculoviralen Konstrukts zur Gewinnung von gereinigtem rekombinantem Fusionsprotein bestehend aus VP22 und der katalytischen Untereinheit der Telomerase

Die Gewinnung des VP22-Telomerase-Fusionsproteins erfolgt in einem Baculo-Expressionssystem, welches zum einen die Ausschleusung des Proteins aus der Zelle und somit dessen native Faltung und Modifikation und zum anderen die affinitätchromatographische Aufreinigung des Proteins erlaubt.

### a. Mutagenese der Plasmide pCMV-VP22-Telo-His und pMelBac(A)

Als Ausgangskonstrukte für die Klonierung stehen die Plasmide 'pCMV-VP22-Telo-His' und der baculovirale Expressionsvektor pMelBac(A) (Invitrogen) zur Verfügung, in denen jeweils über eine 'site-directed PCR mutagenesis' (Kit von Stratagene) eine zusätzliche Hind III-Restriktionsschnittstelle eingefügt wird. Im Falle des Plasmids pCMV-VP22-Telo-His wird dazu der in Tab.8 angegebene Primer #1 verwendet, wodurch neben der zwischen dem CMV-Promotor und der VP22-Sequenz liegenden Hind III-Schnittstelle eine zusätzliche HindIII-Schnittstelle 3'-wärts des 'His-tags' inseriert wird. Im Falle des pMelBac-Vektors wird über die Mutagenese mit Primer #2 (Tab.8) eine zusätzliche Hind III-Schnittstelle- unmittelbar hinter dem Sekretionssignal in die multiple cloning site eingefügt.

**Tab.8: Primer für 'site-directed PCR mutagenesis' zur Insertion zusätzlicher Hind III-Schnittstellen (fett markierte Buchstaben) in die Konstrukte pCMV-VP22-Telo-His (Primer #1) bzw. pMelBac(A)(Primer #2).**

| Primer | Sequenz |
|---|---|
| Primer #1 | 5'-caccattgagtttaaacccgcaagcttgcctcgactgtgccttctagttgc-3' |
| Primer #2 | 5'-tacatttcttacatctatgcgaagctttggggatccgagctcgagatctgc-3' |

### b. Klonierung des Konstrukts pMelBac-VP22-Telo-His

Zur Klonierung des sekretierenden Baculo-Expressionsvektors wird aus dem mutagenisierten Konstrukt pCMV-VP22-Telo-His das Telomerase-haltige Fragment über Restriktionsverdau mit Hind III herausgespalten, isoliert und in den ebenfalls mit Hind III gepaltenen mutagenisierten Vektor pMelBac(A) kloniert. Diejenigen Klone (pMelBac-VP22-Telo-His), bei denen das in pMelBac enthaltenen Honeybee Melittin Sekretionssignal N-terminal mit dem VP22-Telomerase-His-Fragment 'in frame' vorliegt, werden über Restriktionsspaltung und Sequenzierung identifiziert und analysiert.

### Beispiel 10: Klonierung eines baculoviralen Konstrukts zur Gewinnung von gereinigtem rekombinantem Fusionsprotein bestehend aus VP22 und dem SV40 T-Antigen

Die Gewinnung des rekombinanten VP22-Tag-Fusionsproteins erfolgt analog zum VP22-Telomerase-Fusionsprotein in einem sekretierenden Baculo-Expressionssystem. Als Ausgangskonstrukte für die Klonierung des baculoviralen Expressionsvektors stehen die Plasmide pCMV-VP22-TAg und der baculovirale Expressionsvektor pMelBac(A) (Invitrogen) zur Verfügung.

### a. Mutagenese des Konstrukts pCMV-VP22-TAg

Zunächst wird in dem Konstrukt pCMV-VP22-TAg über 'site-directed PCR mutagenesis' (Kit von Stratagene) mit Primer #1 (Tab.9) eine zusätzliche Age 1-Schnittstelle hinter die Sequenz des SV40 T-Antigens bei gleichzeitiger Entfernung des dort vorhandenen Stop-Codons eingefügt. Anschließend wird über eine zweite 'site-directed PCR mutagenesis' mit Primer #2 (Tab.9) eine Bgl II-Schnittstelle zwischen die Sequenzen des CMV-Promotors und des VP22-Proteins inseriert, wobei die dort liegende Hind III-Schnittstelle verlorengeht.

**Tab 9: Primer für 'site-directed PCR mutagenesis' im Vektor pCMV-VP22-Tag zur Insertion einer Age I-Schnittstelle hinter die Sequenz des SV40 T-Antigens (Primer #1) bzw. einer Bgl II-Schnittstellen vor das Start-Codon der VP22-Sequenzen (Primer #2). Die Fett markierten Buchstaben geben die Sequenzen der neu inserierten Restriktionsschnittstellen Age I (Primer #1) bzw. Bgl II (Primer #2) an.**

| Primer | Sequenz |
|---|---|
| Primer #1 | 5'-acacctccccctgaacctgaaacaaccggtgaatgcaattgttgttgttaacgggga-3' |
| Primer #2 | 5'-ggagacccaagctggctagttaagagatctatgacctctcgccgctccgtgaagtcg-3' |

### b. Modifikation des Vektors pMelBac(A)

In den Vektor pMelBac(A) werden 3'-wärts der Honeybee Melittin Sekretionssequenz die beiden Restriktionsschnittstellen Bgl II und Pme I inseriert, indem der Vektor mit Bam HI geöffnet und ein die beiden Restriktionsschnittstellen enthaltendes doppelsträngiges Oligonukleotid einkloniert wird. Die Sequenzen der beiden komplementären Einzelstränge des Oligonukleotids, welche vor Insertion in den pMelBac-Vektor gegeneinander hybridisiert werden, sind in Tabelle 10 angegeben.

**Tab. 10: Die mit Oligo #1 und #2 bezeichneten einzelsträngigen Desoxyoligonukleotide werden vor der Klonierung in den Vektor pMelBac(A) gegeneinander hybridisiert, wobei beiderseits überhängende Enden der Bam HI-Schnittstelle entstehen.**

| Oligo | Sequenz |
|---|---|
| Oligo #1 | 5'-gatccagatctgtttaaacg-3' |
| Oligo #2 | 5'-gatccgtttaaacagatctg-3' |

### c. Klonierung des baculoviralen Expressionsvektors pMelBac-VP22-Tag-His

Durch Herausspalten eines Age I-Fragments aus dem mutagenisierten pCMV-VP22-Tag-Konstrukt und anschließender Re-Ligation wird zunächst die Sequenz des SV40-T-Antigens so in unmitttelbare Nähe des im Konstrukt vorhandenen 'His-tag' gebracht, daß das zu exprimierende VP22-Tag-Fusionsprotein C-terminal mit dem 'His-tag' versehen wird. Anschließend wird aus diesem als pCMV-VP22-Tag-His bezeichneten Konstrukt ein Fragment mit den Restriktionsendonukleasen Bgl II und Pme I herausgeschnitten und dieses gerichtet in den modifizierten und mit den gleichen Restriktionsenzymen gespaltenen Vektor pMelBac kloniert, wodurch der Vektor pMelBac-VP22-Tag-His entsteht.

### Beispiel 11: Gewinnung und Reinigung der VP22-Telomerase-His bzw. VP22-Tag-His-Fusionsproteine

Die Proteinexpression und Reinigung wird nach Anleitung der Firma Invitrogen durchgeführt, wobei die neu konstruierten Vektoren pMelBac-VP22-Tag-His bzw. pMelBac-VP22-Telo-His zur Anwendung kommen.

### Beispiel 12: Identifikation von Peptid- bzw. Antikörper-Liganden zum Transfer von Immortalisierungsproteinen in Kardiomyozyten

Die Identifizierung von Peptid-Liganden, welche mit den zu immortalisierenden Kardiomyozyten interagieren und von diesen internalisiert werden, erfolgt mit Random Peptide Phage Display Bibliotheken der Firma New England Biolabs. Diese enthalten 7mer oder 12mer Peptide in Fusion mit dem P3-Protein von filamentösen Phagen. Die Phagen werden mit den Zellen in Anwesenheit von Chloroquin inkubiert, wobei die Gabe von Chloroquin verhindert, daß in Lysosomen internalisierte Phagen degradiert werden. Nachdem nicht bindende Phagen weggewaschen und Oberflächenassoziierte Phagen durch pH-Änderung von den Zellen abgelöst wurden, werden die internalisierten Phagen durch Lyse der Zellen freigesetzt. Diese Affinitätsselektion wird mehrfach wiederholt ('Panning'). Anschließend werden die relevanten Teile der Phagen-DNA sequenziert und die korrespondierenden Peptid-Motive über kompetitiven ELISA mit entsprechend markierten synthetischen Peptiden auf ihre Bindungsaffinität und Internalisierungsrate überprüft.

Zur Identifizierung von 'Single Chain'-Antikörpern, welche gleichfalls zum Transfer der oben beschriebenen Proteine verwendet werden sollen, werden anstelle der Random Peptide Phage Display Bibliotheken Single Chain Phagemid-Bibliotheken zur Affinitätsselektion herangezogen.

### Beispiel 13: Bereitstellen von organbezogenen Zellen

Als organbezogene Zellen können multipotente Stammzellen verwendet werden, die vor der transienten Immortalisierung und Expansion noch in organspezifische Zellen differenziert werden müssen, oder bereits differenzierte Ausgangszellen des jeweiligen Organes.

Weiter muß zwischen autologen Zellen des jeweiligen Patienten oder allogenen Zellen eines Spenders unterschieden werden.

Als Stammzellen werden mesenchymale Stromazellen des Knochenmarks verwendet. Diese sind in der Lage, sich zu Osteoblasten, Myoblasten, Adipocyten sowie weiteren Zelltypen zu differenzieren. In der Klinik wird Knochenmark routinemäßig unter OP-Bedingungen für die allogene Knochenmarktransplantation gewonnen. Dabei werden jedoch nur die hämatopoetischen Stammzellen benötigt, während die hier interessanten mesenchymalen **Stammzellen als Nebenprodukt entstehen.**

Anderseits können mesenchymale Stammzellen auch aus peripherem Blut gewonnen werden.

Die so gewonnenen Stammzellen werden in konventionellen Zellkulturschalen ausgesät und in alpha-MEM oder IDEM-Medium mit 10 % FKS, sowie Antibiotika wie Penicillin, Streptomycin oder Amphotericin B kultiviert.

Hepatozyten der Leber werden direkt als Primärkultur angelegt. Dopaminerge Ausgangszellen werden im Rahmen einer Organspende entnommen. Herzmuskelzellen können sowohl als Stammzellen aus Knochenmark als auch als Ausgangszellen im Rahmen einer Herzmuskel-Biopsie für die Immortalisierung gewonnen werden.

### Beispiel 14: Expansion und Differenzierung

Ausgangszellen, die bereits terminal differenziert sind, werden in einem üblichen Medium unter Zugabe von Immortalisierungsproteinen oder in Kokultur mit Feederzellen expandiert, ohne daß weitere Maßnahmen erforderlich sind.

Handelt es sich bei zu immortalisierenden Zellen jedoch um replikationsfähige Zellen, bspw. um mesenchymale Stammzellen aus dem Knochenmark, ist zunächst durch Zugabe von Differenzierungssubstanzen eine Differenzierung in die organspezifischen Zellen erforderlich, bevor die transiente Immortalisierung erfolgen kann.

Durch Behandlung mit 5'-Azacytidin können mesenchymale Stammzellen z.B. zu kardiomyogenen Zellen differenziert werden; Makino et al, J.Clin.Invest. 1999,103:697-705. Eine 5'-Azacytidin-Behandlung von Stammzellen, die das Entwicklungspotential von Herzmuskelzellen haben, löst Differenzierungsvorgänge durch Demethylierung aus. Sehr wahrscheinlich wird dabei der Promotor von noch unbekannten essentiellen Herzmuskel-Differenzierungsgenen aktiviert.

Nach Erkenntnis der Erfinder hat 5'-Azacytidin aber ein mutagenes Potential. Daher wird die Differenzierung von Stammzellen zu Herzmuskelzellen erfindungsgemäß durch Gabe zumindest einer weiteren Differenzierungssubstanz verbessert. Hierzu ist die Substanz Trichstatin A (TSA) vorgesehen. TSA bewirkt eine Inhibition der Histon-Deacetylierung. Diese Histon-Deacetylierung steht im Zusammenhang mit transkriptioneller Repression von CpG-Methylierungen.

CpG-Inseln, also Bereiche mit mehreren CpG-Dinukleotiden, finden sich vorwiegend in Promotoren. Durch die Methylierungen kann die Aktivität eines CpG-reichen Promotors erheblich inhibiert werden. Dies erfolgt z.B. wenn 5'-Azacytidin in die DNA replizierender Zellen eingebaut wird, weil aufgrund der Aza-Gruppe an Position 5 dort keine Methylierung durch zelluläre Prozesse erfolgen kann.

Eine Kombination von 5'-Azacytidin und TSA kann somit synergistisch wirken, wie in Tumorzellen bereits gezeigt wurde; siehe Cameron et al., "Synergy of demethylation and histone deacetylase inhibition in the re-expression of genes silenced in cancer", Nat.Genet. Band 21, Seiten 103-107.

Dieser Synergismus wird erfindungsgemäß auf die Differenzierung von Stammzellen zu Herzmuskelzellen übertragen. Durch die weitere Zugabe von all-trans-Retinsäure und Amphotericin B wird die Differenzierung weiter optimiert. Retinsäure ist eine Differenzierungssubstanz, die in der Myoblasten-Zellinie H9C2 einen Herzmuskelphenotyp versus eines Skelettmuskelphenotyps begünstigt; siehe Menard et al., "Modulation of L-type calcium channel expression during retinoic acid-induced differentiation of H9C2 cardiac cells", J.Biol.Chem. Band 274, Seiten 29063-29070.

Auch Amphotericin B kann die Differenzierung in Richtung Herzmuskelzellen günstig beeinflussen; siehe Phinney et al. "Plastic adherent stromal cells from the bone marrow of commonly used strains of inbred mice: variations in yield, growth, and differentiation", J.Cell.Biochem. Band 72, Seiten 570-585.

Der Vorteil der Verwendung einer Kombination aus mehreren Differenzierungssubstanzen besteht darin, daß synergistische Effekte erzielt werden, die die mutagene Wirkung von 5'-Azacytidin erheblich reduzieren oder gar aufheben. Dies ist für die spätere klinische Anwendung der aus Stammzellen gewonnenen Herzmuskelzellen von ausschlaggebender Bedeutung.

Wenn die Differenzierungssubstanz Dexamethason, Conget et al. a.a.O. alleine oder in Kombination mit den oben beschrieben vier Differenzierungssubstanzen verwendet wird, können Stammzellen zu Knochen- und Knorpelzellen differenziert werden.

Die so differenzierten Zellen werden dann zur weiteren Expansion transient immortalisiert.

### Beispiel 15: Transplantation

Nach erfolgter Remortalisierung und entsprechender Qualitätskontrolle werden die Zellen mit konventionellen Techniken in die geschädigten Organe transplantiert, z.B. mit einer Spritze in das Organ injiziert. Dies kann wiederholt geschehen, da aufgrund der Immortalisierung Material in jeder gewünschten Menge zur Verfügung steht.

Ohne die Immortalisierung könnten aus einer Stammzelle nur ca. 5 x 10⁸ bis 1 x 10⁹, bei einem älteren Spender u.U. sogar nur noch 1 x 10⁶ Zellen entstehen. Dies würde für eine Regeneration wahrscheinlich zu wenig sein. Bei autologer Transplantation muß der Patient ferner so lange warten, bis sich die Zellen zur erforderlichen Zellzahl vermehrt haben. Bei allogener Transplantation kann dagegen jederzeit die gewünschte Zellzahl bereitgestellt werden.

In besonderen Notfällen ist es sinnvoll, erst eine allogene Transplantation durchzuführen, um später auf autologe Transplantation umzusteigen.

## Patentansprüche

1. in vitro Verfahren zur transienten Immortalisierung von Zellen, **dadurch gekennzeichnet, dass** ein Immortalisierungsprotein mittels Nanopartikel oder Liposomen in die Zellen eingebracht wird.

2. Verfahren zur transienten Immortalisierung von Zellen, nach Anspruch 1 **dadurch gekennzeichnet, dass** die Nanopartikel auf der Oberfläche modifiziert sind, insbesondere Liganden oder Single-Chain Antikörper aufweisen.

3. Verfahren zur transienten Immortalisierung von Zellen nach einem der Ansprüche 1 bis 2, wobei die Immortalisierungsproteine Transformationsproteine sind.

4. Verfahren zur transienten Immortalisierung von Zellen nach Anspruch 3, wobei die Transformationsproteine Expressionsprodukte viraler Oncogene, vorzugsweise von SV40 Tag, HPV E6, HPV E7, Adenovirus E1A, AdenovirusE1B, und/oder zelluläre Oncogene, vorzugsweise ras, myc, src, besonders vorzugsweise SV40Tag sind.

5. Verfahren zur transienten Immortalisierung von Zellen nach einem der Ansprüche 1 bis 2, wobei Immortalisierungsproteine Telomerproteine sind.

6. Verfahren zur transienten Immortalisierung von Zellen nach Anspruch 5, bei dem als Telomerprotein die katalytische Untereinheit, vorzugsweise hTRT^{plus} (DSM 14569), der humanen Telomerase verwendet wird.

7. Verfahren zur Gewinnung von Zellen, mit den Schritten:
(a) Bereitstellen von organbezogenen Zellen,
(b) Transientes Immortalisieren der organbezogenen Zellen durch externe Zufuhr von Immortalisierungsprotein mittels Nanopartikel oder Liposomen nach einem der Ansprüche 1 bis 6,
(c) Expansion der immortalisierten Zellen und Remortalisierung der expandierten Zellen durch Beenden der Zufuhr von Immortalisierungsprotein aus (b).

8. Verfahren nach Anspruch 7, **dadurch gekennzeichnet, dass** als organbezogene Zellen multipotente Stammzellen, vorzugsweise mesenchymale Stromazellen des Knochenmarks, verwendet werden.

9. Verfahren nach Anspruch 7 oder 8, **dadurch gekennzeichnet, dass** als organbezogene Zellen seilende oder ruhende, terminal differenzierte Ausgangszellen des Organs, vorzugsweise Herzmuskelzellen, verwendet werden.

10. Verfahren nach einem der Ansprüche 7 bis 9, **dadurch gekennzeichnet, dass** als organbezogene Zellen autologe Zellen verwendet werden.

11. Verfahren nach einem der Ansprüche 7 bis 9, **dadurch gekennzeichnet, dass** als organbezogene Zellen allogene Zellen verwendet werden.

12. Verfahren nach einem des Ansprüche 7-11, **dadurch gekennzeichnet, dass** aus den expandierten Zellen ein Transplantat für die Regeneration eines Organs hergestellt wird.

## Claims

1. An in-vitro method for the transient immortalization of cells, **characterized in that** an immortalizing protein is introduced into the cells by way of nanoparticles or liposomes.

2. A method for the transient immortalization of cells according to claim 1, **characterized in that** the nanoparticles are modified on the surface, in particular that they comprise ligands or single-chain antibodies.

3. A method for the transient immortalization of cells according to any one of claims 1 to 2, wherein the immortalizing proteins are transformation proteins.

4. The method for the transient immortalization of cells according to claim 3, wherein the transformation proteins are expression products of viral oncogenes, preferably of SV40 Tag, HPV E6, HPV E7, adenovirus E1A, adenovirus E1B and/or cellular oncogenes, preferably ras, myc, src, with SV40 Tag being particularly preferred.

5. A method for the transient immortalization of cells according to any one of claims 1 to 2, wherein the immortalizing proteins are telomere proteins.

6. The method for the transient immortalization of cells according to claim 5, wherein the catalytic subunit, preferably hTRT^{plus} (DSM 14569) of human telomerase is used as the telomere protein.

7. A method for obtaining cells, comprising the following steps:
(a) providing organ-related cells,
(b) transiently immortalizing the organ-related cells by the external supply of immortalizing protein by way of nanoparticles or liposomes according to any one of claims 1 to 6,
(c) expanding the immortalized cells and remortalizing the expanded cells by terminating the supply of immortalizing protein from (b).

8. The method according to claim 7, **characterized in that** multipotent stem cells, preferably mesenchymal stromal cells of the bone marrow, are used as organ-related cells.

9. The method according to claim 7 or 8, **characterized in that** dividing or resting, terminally differentiated original cells of the organ, preferably cardiac muscle cells, are used as organ-related cells.

10. A method according to any one of claims 7 to 9, **characterized in that** autologous cells are used as the organ-related cells.

11. A method according to any one of claims 7 to 9, **characterized in that** allogenous cells are used as the organ-related cells.

12. A method according to any one of claims 7 to 11, **characterized in that** a transplant is produced from the expanded cells for regenerating an organ.

## Revendications

1. - Procédé *in vitro* d'immortalisation transitoire de cellules, **caractérisé par le fait qu'**une protéine d'immortalisation est introduite dans les cellules au moyen de nanoparticules ou de liposomes.

2. - Procédé d'immortalisation transitoire de cellules selon la revendication 1, **caractérisé par le fait que** les nanoparticules sont modifiées sur leur surface, et comprennent en particulier des ligands ou des anticorps à chaîne unique.

3. - Procédé d'immortalisation transitoire de cellules selon l'une des revendications 1 à 2, dans lequel les protéines d'immortalisation sont des protéines de transformation.

4. - Procédé d'immortalisation transitoire de cellules selon la revendication 3, dans lequel les protéines de transformation sont des produits d'expression d'oncogènes viraux, de préférence SV40 Tag, HPV E6, HPV E7, Adénovirus E1A, Adénovirus E1B, et/ou d'oncogènes cellulaires, de préférence ras, myc, src, de façon particulièrement préférée sV40Tag.

5. - Procédé d'immortalisation transitoire de cellules selon l'une des revendications 1 à 2, dans lequel les protéines d'immortalisation sont des protéines télomériques.

6. - Procédé d'immortalisation transitoire de cellules selon la revendication 5, dans lequel la sous unité catalytique de la télomérase humaine, de préférence hTRT^{plus} (DSM 14569), est utilisée en tant que protéine télomérique.

7. - Procédé d'obtention de cellules, comprenant les étapes de :
(a) apport de cellules associées à un organe,
(b) immortalisation transitoire des cellules associées à un organe par apport exogène d'une protéine d'immortalisation au moyen de nanoparticules ou de liposomes selon l'une quelconque des revendications 1 à 6,
(c) développement des cellules immortalisées et remortalisation des cellules développées par arrêt de l'apport de la protéine d'immortalisation selon (b).

8. - Procédé selon la revendication 7, **caractérisé par le fait que** des cellules souches multipotentes, de préférence des cellules mésenchymateuses du stroma de la moelle osseuse sont utilisées comme cellules associées à un organe.

9. - Procédé selon l'une des revendications 7 ou 8, **caractérisé par le fait que**, comme cellules associées à un organe, sont utilisées des cellules de départ différenciées de façon terminale en division ou quiescentes de l'organe, de préférence des cellules de myocarde.

10. - Procédé selon l'une des revendications 7 à 9, **caractérisé par le fait que** des cellules autologues sont utilisées comme cellules associées à un organe.

11. - Procédé selon l'une des revendications 7 à 9, **caractérisé par le fait que** des cellules allogéniques sont utilisées comme cellules associées à un organe.

12. - Procédé selon l'une des revendications 7 à 11, **caractérisé par le fait qu'**un greffon est produit à partir des cellules développées pour la régénération d'un organe.
